# EUROPEAN PATENT APPLICATION

(11) **EP 0 947 167 A1**
(43) Date of publication of application: **06.10.1999**
(21) Application number: 99302434.8
(22) Date of filing: 29.03.1999
(51) Int. Cl.: A61B 17/00, A61B 17/32

(54) **Methods and apparatus to recognize surgical apparatus**

(30) Priority: 30.03.1998 US 50755
(71) Applicant: ETHICON ENDO-SURGERY, INC., Cincinnati, Ohio 45242 (US)
(72) Inventor: Houser, Kevin, Centerville, Ohio 45459 (US)
(74) Representative: Mercer, Christopher Paul

(57) **Abstract**

The present invention relates to method and apparatus to detect or identify surgical instruments. One apparatus includes apparatus to detect a surgical apparatus comprising a receptacle assembly configured to receive a plug assembly of the surgical apparatus. At least one switching device is coupled to the receptacle assembly. Circuitry is responsive to the switching device to detect the state of the switching device. One method in accordance with the present invention comprising the steps of sensing the condition of a switch and inserting a connector of an ultrasonic surgical instrument into a connector of a generator unit. The method also includes the steps of sensing the condition of a switch after the connector is inserted and determining whether to provide power to the ultrasonic surgical apparatus.

## Description

### TECHNICAL FIELD

The present invention relates generally to detection apparatus, and more particularly to detection apparatus to detect or recognize surgical apparatus connected to a power supply unit.

### BACKGROUND OF THE INVENTION

Ultrasonic surgical instruments have been developed to perform various surgical procedures and operations, such as cutting and coagulation tissues. For example, U.S. Patent Nos. 5,263,957 and 5,324,299, hereby incorporated by reference, disclose ultrasonic surgical scalpels, and U.S. Patent No. 5,449,370, hereby incorporated by reference, describes an ultrasonic trocar apparatus. Ultrasonic instruments have also been developed to perform various endoscopic and laparoscopic procedures. For instance, U.S. Patent No. 5,322,055, hereby incorporated by reference, discloses an ultrasonic clamp coagulator apparatus for laparoscopic and endoscopic use.

Each of these ultrasonic surgical instruments usually has a handpiece assembly and surgical attachment or accessory. The handpiece assembly typically carries an ultrasonic transducer to convert electric energy into mechanical motion. The transducer of the surgical instruments is usually energized with electrical signals from an ultrasonic generator unit.

The generator unit provides power to energize the transducer of the handpiece assembly at a certain frequency. The power from the generator unit is usually transmitted though a cable assembly to the handpiece assembly. The cable assembly typically includes a male plug that can be removably attached to a female receptacle on a front panel of the generator unit.

The generator unit can be utilized to provide power to a number of different ultrasonic handpiece assemblies. However, the generator unit usually cannot "recognize" or "identify" the handpiece assembly that is attached to the generator unit to change the power requirements for a particular handpiece assembly, or to prevent undesirable or unsuitable handpiece assemblies from being utilized with the generator unit.

### SUMMARY OF THE INVENTION

In view of the above, the present invention provides apparatus and methods to recognize and/or identify (that is, identify the compatibility and characteristic of) various types of surgical devices, such as handpiece assemblies and drive units. The apparatus of the present invention can be easily installed or incorporated into existing or new surgical devices and generator units, and are relatively inexpensive.

The apparatus and methods provide detection apparatus to recognize when a surgical device is attached to the generator unit or power supply unit. Once the detection apparatus identifies the surgical device, the generator unit can provide power to energize the surgical device. The power can also be altered, set, and/or adjusted to meet various requirements of the surgical device. The detection apparatus can further allow the generator unit to lock-out undesirable or unsuitable surgical devices and can notify the user that the surgical devices cannot be operated with the generator unit.

One apparatus in accordance with the present invention includes apparatus to detect a surgical apparatus comprising a receptacle assembly configured to receive a plug assembly of the surgical apparatus. At least one switching device is coupled to the receptacle assembly. Circuitry is responsive to the switching device to detect the state of the switching device.

One method in accordance with the present invention comprises the steps of sensing the condition of a switch, and inserting a connector of an ultrasonic surgical instrument into a connector of a generator unit. The method also includes the steps of sensing the condition of a switch after the connector is inserted, and determining whether to provide power to the ultrasonic surgical apparatus.

The invention, together with attendant features and advantages, will best be understood by reference to the following detailed description of the preferred embodiments of the invention, taken in conjunction with the accompanying drawings. It is to be understood that both the foregoing general description and the following detailed description are exemplary and explanatory and are intended to provide further explanation of the invention as claimed.

### BRIEF DESCRIPTION OF THE DRAWINGS

FIG. 1 is an elevational view of an ultrasonic surgical system;
FIG. 2 is a perspective view of a receptacle assembly and a plug assembly of the surgical system of FIG. 1 in accordance with the present invention;
FIG. 3 is a side view of the receptacle assembly and the plug assembly of FIG. 2;
FIG. 4 is a side view of the plug assembly inserted into the receptacle assembly ofFIG. 2;
FIG. 5 is a schematic diagram of circuitry of the surgical system of FIG. 1;
FIG. 6 is schematic diagram of alternative circuitry of the surgical system of FIG. 1;
FIG. 7 is a schematic diagram of a control system of the surgical system of FIG. 1;
FIG. 8 is a perspective view of another receptacle assembly and plug assembly of a surgical system in accordance with the present invention;
FIG. 9 is a perspective view of another receptacle assembly and plug assembly of a surgical system in accordance with the present invention;
FIG. 10 is a perspective view of another receptacle assembly and plug assembly of a surgical system in accordance with the present invention;
FIG. 11 is a perspective view of another receptacle assembly and plug assembly of a surgical system in accordance with the present invention; and
FIG. 12 is a perspective view of a handpiece assembly and a blade and adaptor assembly of a surgical system in accordance with the present invention.

### DESCRIPTION OF THE PREFERRED EMBODIMENTS

While the present invention is susceptible of embodiments in various forms, there is shown in the drawings and will hereinafter be described a presently preferred embodiment, with the understanding that the present disclosure is to be considered as an exemplification of the invention, and is not intended to limit the invention to the specific embodiment illustrated.

The present invention is particularly directed to methods and apparatus to detect a handpiece assembly or a surgical apparatus connected to a power supply unit or generator unit. When the system detects the handpiece assembly, the system can provide power to the handpiece assembly. The system may also recognize or identify the type or model of the handpiece assembly and can alter, set, and/or adjust various parameters, functions, and outputs to meet the requirements of the handpiece assembly. The system can further lock-out undesirable handpiece assemblies and notify the user that the handpiece assemblies cannot be operated with the system.

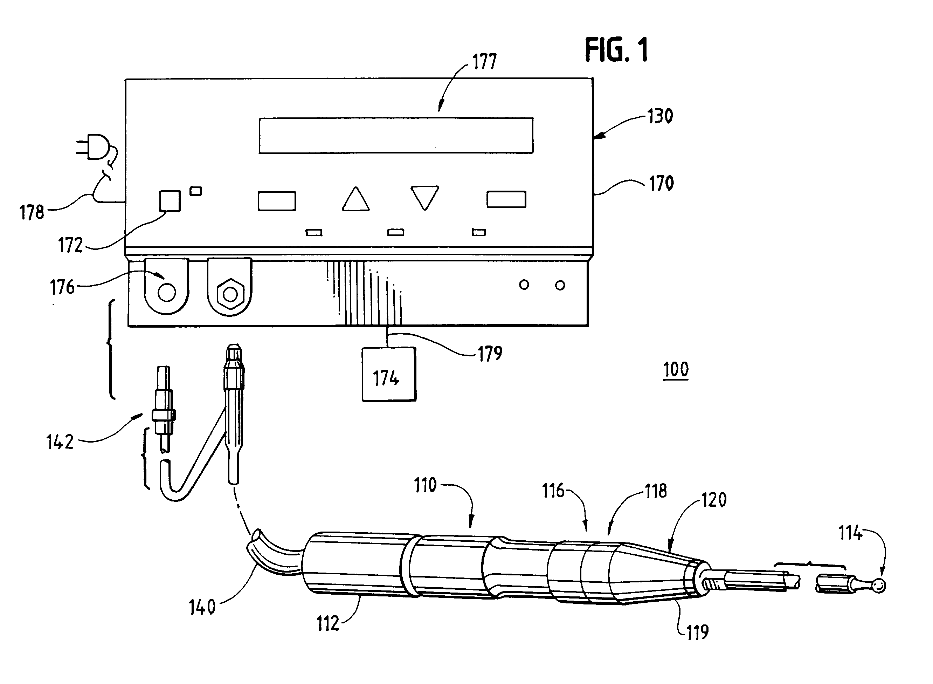

Referring now to the drawings, and more particular to FIG. 1, a presently preferred embodiment of a surgical system, generally designated 100, is illustrated. The surgical system 100 preferably includes a handpiece assembly or an ultrasonic surgical drive unit 110, a surgical instrument or accessory, a power supply unit or generator unit, and detection apparatus. The detection apparatus, as further described below, is employed to detect or recognize the handpiece assembly when connected to the generator unit.

The handpiece assembly 110 of the surgical system preferably includes a housing 112 adapted to isolate the operator from a drive member (not shown), such as, for example, a transducer assembly, a motor, a power oscillator, or the like. The drive member is preferably carried by the handpiece assembly 110 to convert electrical energy from the generator unit into mechanical motion to energize or vibrate an end effector 114 for cutting, cauterizing, and/or coagulation of tissues. It is also contemplated that the drive member may be a laser source, a power supply for electro-cauterization, or any other similar device.

The distal end 116 of the handpiece assembly 110 preferably receives and is fitted to the proximal end 118 of the surgical instrument 120 by insertion of the handpiece assembly 110 into the housing 119 of the surgical instrument 120. The handpiece assembly 110 is configured to receive a variety of surgical instruments to perform various procedures and operations (i.e., clamp coagulation devices, surgical scalpels, trocars, etc.). The surgical instrument 120 is preferably attached to and removed from the handpiece assembly 110 as a unit. The surgical instrument 120 can be configured for disposal after a single use, or for reuse, including limited reuse, and can be any suitable surgical device.

The generator unit 130 of the surgical system 100 sends electrical signals through a cable assembly 140 to activate or energize the drive member of the handpiece assembly 110. The cable assembly 140 is preferably fixedly attached to the handpiece assembly 110 at one end and has a connector or a plug assembly 142 at the other end. The cable assembly 140 contains at least one pair of electrical conductors to carry electrical signals from the generator unit 130 to the drive member of the handpiece assembly 110. The cable assembly 140 is preferably a shielded cable, and may comprise a coaxial cable or any other suitable type of cable.

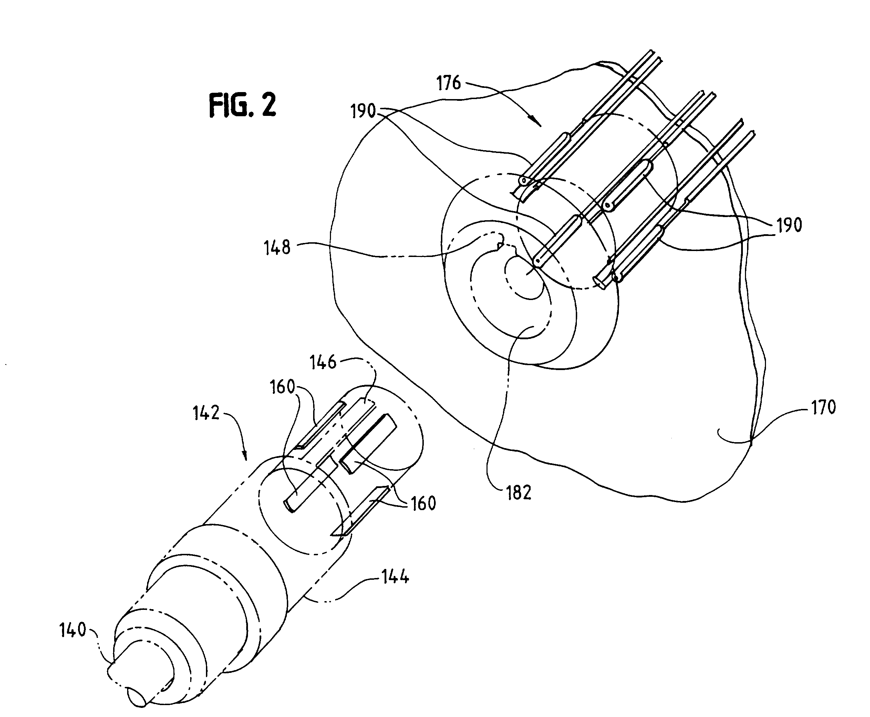

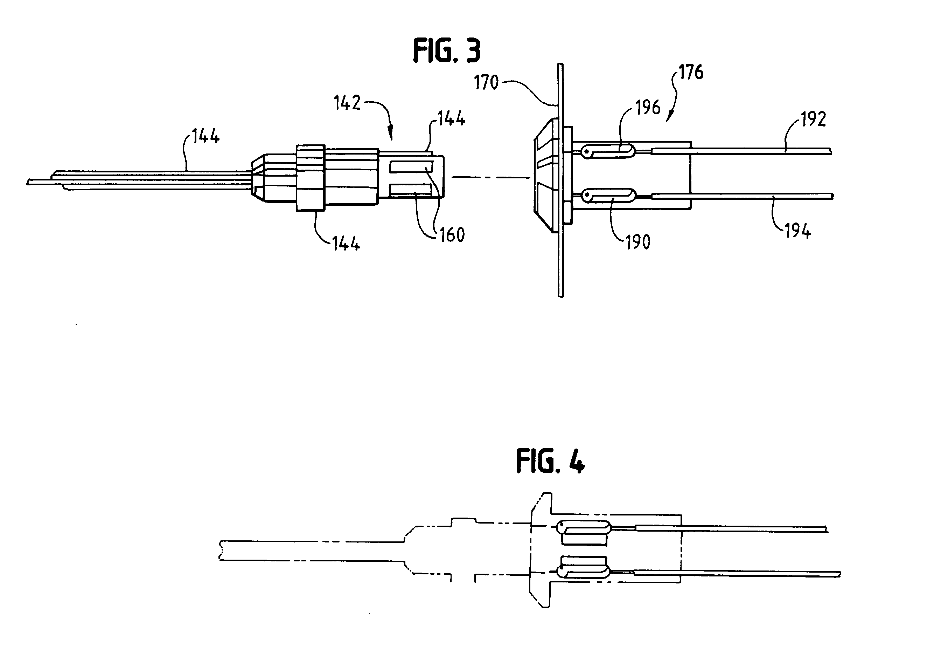

Referring now to FIGS. 2 and 3, the plug assembly 142 of the cable assembly 140 is preferably a male plug assembly 144 that can be inserted into a receptacle assembly or connector of the generator unit 130, as further described below. It is contemplated that the plug assembly 142 may be any suitable device that can be connected to the generator unit 130. The plug assembly 142 preferably includes a key connector 146 to fit with a key slot 148 of the receptacle assembly. The key connector 146 and key slot 148 properly align and hold the plug assembly 142 in a fixed or predetermined position with respect to the receptacle assembly as shown in FIG. 4.

As shown in FIGS. 2 and 3, one or more actuating devices 160 (four being shown) are attached or coupled to the plug assembly 142. The actuating devices 160 are preferably permanent magnets. It is contemplated that the actuating devices 160 can be any suitable device, such as, for example, electro-magnets, to activate a sensor or switch. The actuating devices 160 are positioned on the outer circumference of the plug assembly 142 and are arranged in a predetermined pattern or position on the plug assembly 142. It will be recognized that the actuating devices 160 can be arranged in various patterns depending upon the type of handpiece assembly 110. It is also contemplated that any suitable number (i.e., one or more) of actuating devices may be arranged on the plug assembly 142 in any suitable pattern.

When the handpiece assembly 110 of the surgical system 100 is connected to the generator unit 130, the handpiece assembly 110 can be recognized or detected by the system 100. Once the handpiece assembly 110 is recognized, the generator unit 130 can then provide the suitable power to the handpiece assembly 110. However, undesirable or unsuitable handpiece assemblies can be locked-out or otherwise prevented from being used with the generator unit 130. When the system does not recognize the handpiece assembly 110 or determines that the handpiece assembly 110 is undesirable, the system 100 may activate an indicator device (i.e., a light emitting diode (LED) or an alarm) and/or may provide information to indicate that the handpiece assembly 110 cannot be used with the generator unit 130.

The generator unit 130 preferably includes a housing 170, a control system integral with the generator, a power switch 172, a triggering mechanism 174, the receptacle assembly 176, and an output device 177. The generator unit 130 also has a power line 178 for insertion in an electro-surgical unit or a conventional electrical outlet. It is contemplated that the generator unit 130 can also be powered by a direct current (DC) source, such as a battery.

The housing 170 of the generator unit 130 preferably has a rectangular configuration and is constructed from polymeric and metallic materials. As those skilled in the art will recognize, the size and shape of the housing 170 may be any suitable size and configuration, and the housing 170 may be made from a variety of materials without departing from the spirit and scope of the invention.

The power switch 172 and triggering mechanism 174 of the generator unit 130 are preferably mounted to the front panel of the housing 170. The power switch 172 controls the electrical power provided to the generator unit 130, and the triggering mechanism 174 of the generator unit 130 allows a user to activate the generator unit 130 so that electrical energy may be continuously supplied to the drive member of the handpiece assembly 110. The triggering mechanism 174 preferably comprises a foot activating switch that is detachably coupled or attached to the generator unit 130 by a cable or cord 179.

Alternatively, the triggering mechanism 174 can be configured as a hand switch incorporated into the handpiece assembly 110 to allow the generator unit 130 to be activated by the user. When the generator unit 130 is activated via the triggering mechanism 174, electrical energy is applied by the generator unit 130 to the drive member of the handpiece assembly 110.

The output device 177 of the generator unit 130 is also preferably mounted to the front panel of the housing 170. The output device 177 is preferably a liquid crystal display to provide various information to the user, but can be any suitable device. The output device 177 is preferably linked or coupled to the control system of the generator unit 130 as further described below.

As shown in FIG. 1, the receptacle assembly 176 of the generator unit 130 is mounted to the front panel of the housing 170. The receptacle assembly 176 preferably has a cavity 182, such as a socket, to receive the plug assembly 142. The receptacle assembly 176 is preferably a female receptacle assembly. It will be recognized that the receptacle assembly 176 may be any suitable connector.

As shown in FIGS. 2 and 3, one or more sensors or switches 190 (four being shown) are coupled to the receptacle assembly 176. The switches 190 are preferably magnetically operated switches or reed switches that are designed to change states when a magnetic field is applied to the switches 190. The reed switches preferably include a pair of flexible reed contacts which are fabricated from electrically conductive material. It is contemplated that the switches 190 may be any suitable proximity switch (i.e., a hall effect switch, a light emitting diode, a photo-detector, etc.), any suitable mechanical switch (i.e., a micro--switch, a membrane switch, etc.) or any other suitable sensor or switch.

The switches 190 are preferably mounted to the outer circumference of the receptacle assembly 176 in a predetermined pattern or position. The switches 190 can change states or conditions upon the presence or absence of the actuating devices 160. The switches 190 are electrically connected to wires or conductors 192 and 194. The conductors extend from the switches to the control system of the generator unit 130 as further described below.

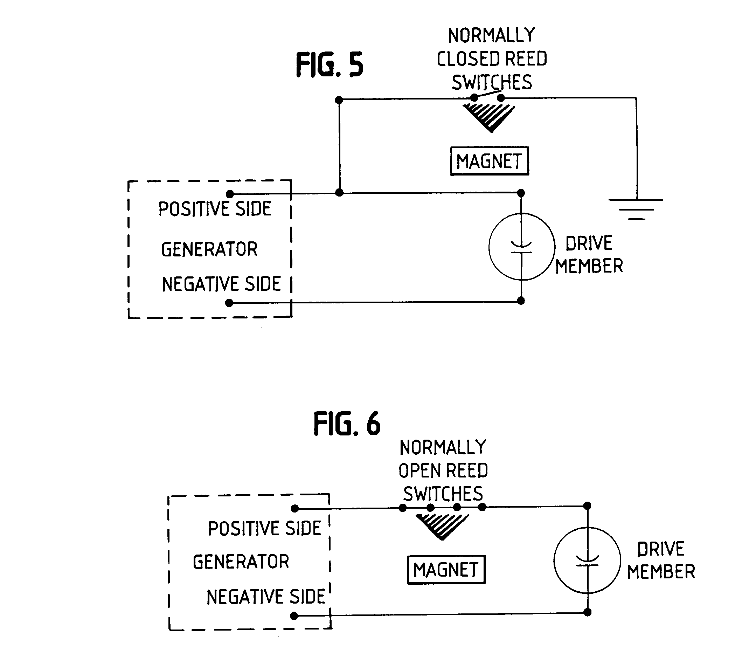

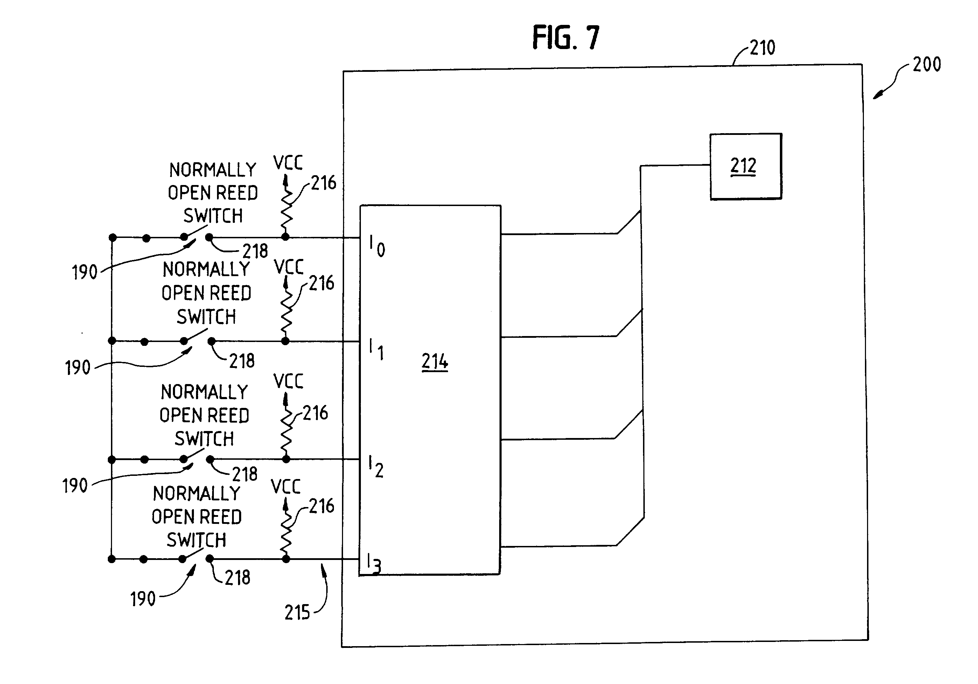

When the plug assembly 142 is inserted into the receptacle assembly 176 of the generator unit 130, the actuating devices 160 of the plug assembly 142 are brought into close proximity to one or more of the switches 190 to change the switches 190 from a first condition or state (i.e., a closed circuit) to a second condition or state (i.e., an open circuit). Preferably, the switches 190 open to form an opened circuit between the control system of the generator unit 130 and the drive member of the handpiece assembly 110 as shown in FIG. 5. When the plug assembly 142 is removed from the receptacle assembly 176, the switches 190 close forming a closed circuit. It will be recognized that the switches 190 may be normally opened switches so that when the plug assembly 142 is inserted into the receptacle assembly 176, the switches 190 close to allow energy from the generator unit 130 to be provided to the drive member of the handpiece assembly 110 as shown in FIG. 6. Thus, when the plug assembly 142 is removed from the receptacle assembly 176, the switches 190 open. It will also be recognized that the switches are preferably arranged in parallel.

Referring now to FIG. 7, a schematic diagram of the control system 200 of the generator unit 130 is illustrated. The control system 200 preferably includes a control unit 210 to sense or detect the state of the switches. The control unit 210 preferably includes a controller 212 having an input port or decoder 214. An exemplary control system of a generator unit 130 is disclosed in U.S. Patent No. 5,026,387, which is herein incorporated by reference.

The decoder 214 preferably detects the state of the switches and provides output signals to the control unit over lines or bus 215. Although a four bit binary decoder is illustrated, it will be recognized that the decoder device may have any suitable number of inputs and outputs (i.e. one or more).

Each of the inputs of the decoder 214 are preferably electrically connected to contact 218 of the switches 190 and are also connected to a high level voltage (i.e., + 5 volts) through a resistor 216. The contact 220 of the switches 190 are connected to ground. The inputs of the decoder 214 are preferably electrically connected with the switches 190 in parallel. It is also contemplated that the decoder 214 may be a discrete component. The control unit 210 can continuously monitor or scan the outputs of the decoder 214 in any combination at a preselected frequency and duration to sense or determine the state or condition of the switches 190.

The state of the switches 190 can be used to detect that a handpiece assembly 110 is connected to the generator unit 130. When the plug assembly 142 is connected to the receptacle assembly 176, the control unit 220 preferably detects if one or more switches 190 have closed. When the switches 190 are closed, the outputs of the decoder 214 will be low, and when the switches 190 are opened, the outputs of the decoder 24 will be high. Upon detection of one or more closed switches 190, the generator unit 130 can provide appropriate power to the handpiece assembly 110. However, when the appropriate switches 190 are not closed, the generator unit 130 will not provide power to the handpiece assembly 110 and the generator unit 130 may display a status message on the output device 177. The generator unit 130 may also activate an indicator device (i.e., an audio or visual device).

The control unit 210 may further decode the switches 190 to determine or identify the type or model of the handpiece assembly 110 or surgical instrument 150 connected to the generator unit 130 in order to set, adjust, and/or alter signals and/or power provided to the handpiece assembly 110. The particular combination of high and low signals at the input of the decoder 214 will indicate the type of handpiece assembly 110 being used. For example, the coding of the decoder 214 could be coded as 0010 and corresponded to a particular type handpiece assembly. Upon recognition of the handpiece assembly, the generator unit 130 can provide appropriate power to the handpiece assembly 110.

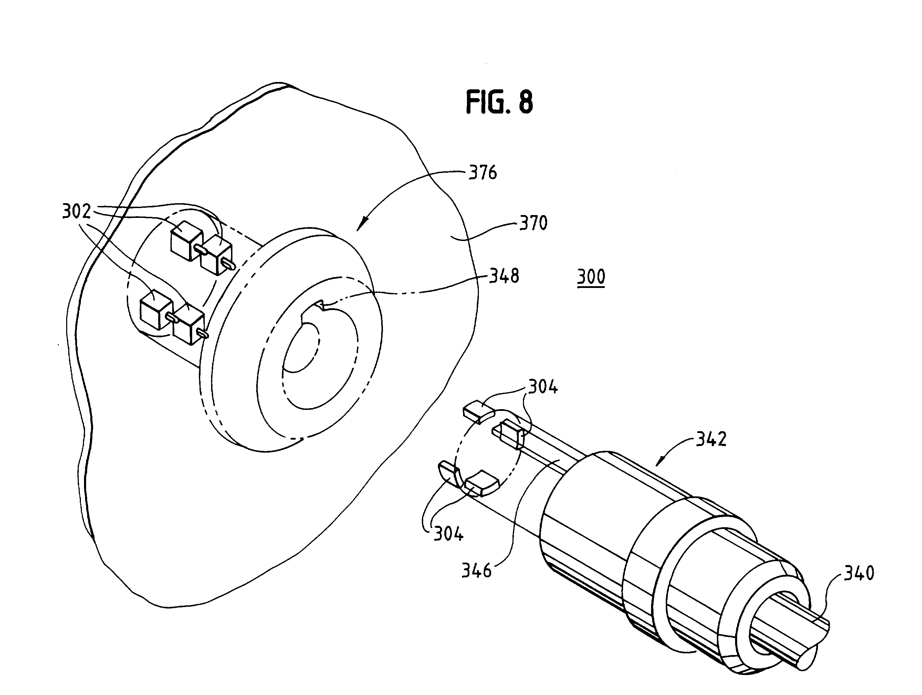

Referring now to FIG. 8, another embodiment of detection or identification apparatus of a surgical system 300 is illustrated. The surgical system 300 in many respects corresponds in construction and function to the surgical system 100 of FIG. 1 except that one or more mechanical switches 302 are located at predetermined positions on the receptacle assembly 376 and one or more protuberances 304 are located at predetermined positions on the plug assembly. Components of the present surgical system 300 which generally correspond to those components of the surgical system 100 of FIG. 1 are designated by like-numerals in the three hundred series.

The detection apparatus of the surgical system 300 can detect when the plug assembly 342 is attached to the receptacle assembly 376 of the generator unit 330 to detect or recognize the type or model of the handpiece assembly 310. When the plug assembly 342 is inserted into receptacle assembly 376, one or more protuberances 304 are brought into close proximity with of one or more of the mechanical switches 302 to alter or change the mechanical switches from a first condition to a second condition. The change in condition of the switches 302 is detected by the control system of the generator unit 330 to provide appropriate power to the handpiece assembly 310 or to lock-out the handpiece assembly 310 as described above.

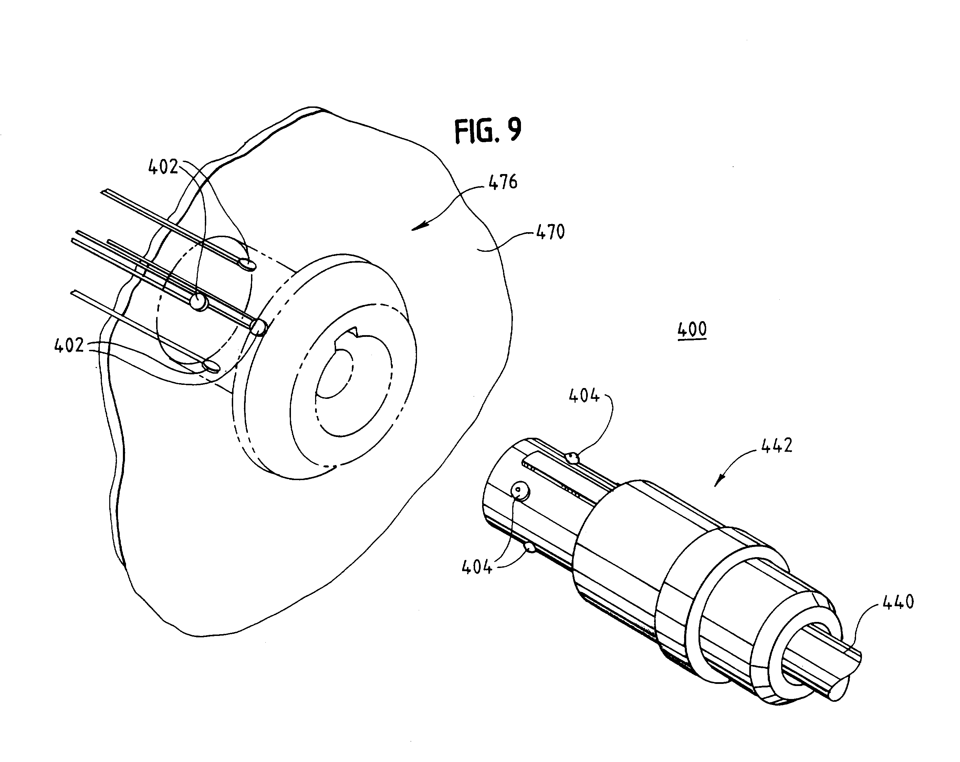

Referring now to FIG. 9, another embodiment of detection or identification apparatus of a surgical system 400 is illustrated. The surgical system 400 in many respects corresponds in construction and function to the surgical system 100 of FIG. 100 except that one or more membrane switches 402 are arranged at predetermined positions on the receptacle assembly 476 and one or more protuberances 404 are arranged at predetermined positions on the plug assembly 442. Components of the present surgical system 400 which generally correspond to the components of the surgical system 100 of FIG. 1 are designated by like-numbers in the four-hundred series.

The detection apparatus of the surgical system 400 detects when the plug assembly 442 is attached to the receptacle assembly 476 of the generator unit 430 to detect or identify the type or model of handpiece assembly 410. When the plug assembly 442 is inserted into receptacle assembly 476, one or more protuberances 404 are brought into close proximity with of one or more of the membrane switches 402 to cause the protuberances 404 to momentarily or continuously alter or change the membrane switch 402 from a first condition to a second condition. The change in condition of the switches 402 is detected by the control system of the generator unit 430 to provide appropriate power to the handpiece assembly 410 or to lock-out the handpiece assembly 410 as described above.

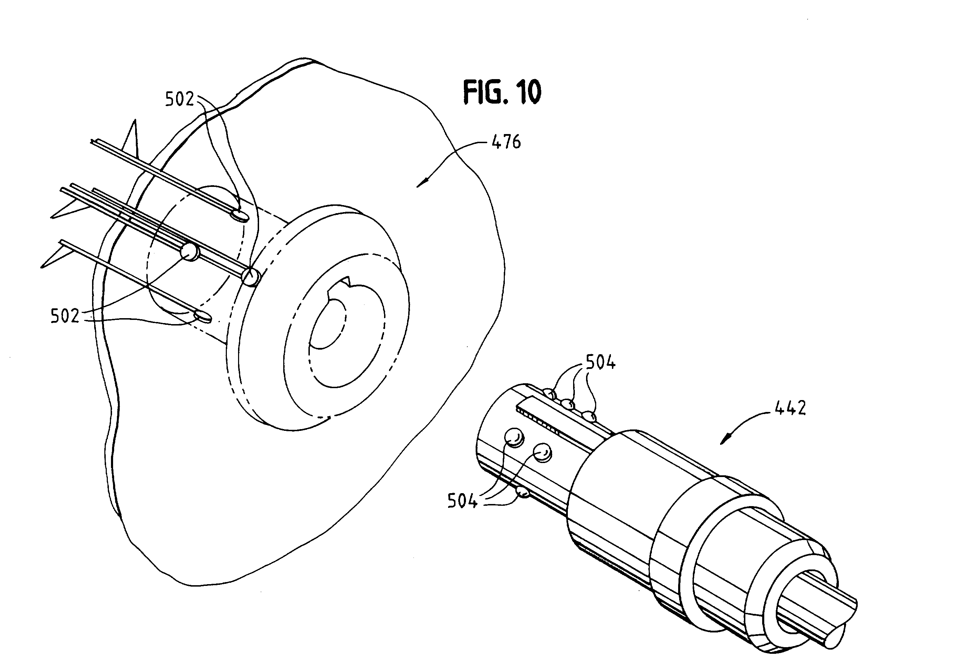

As shown in Figure 10, a plurality of protuberances 504 may be positioned axially along the plug assembly 442 such that the membrane switches 502 of the receptacle assembly 472 will alter conditions sequentially a predefined number of times corresponding to the number of protuberances. This alteration of membrane switch condition may be utilized by the control system of the generator unit 130 to detect and/or identify the handpiece assembly 110 of the surgical apparatus connected to the generator unit 130.

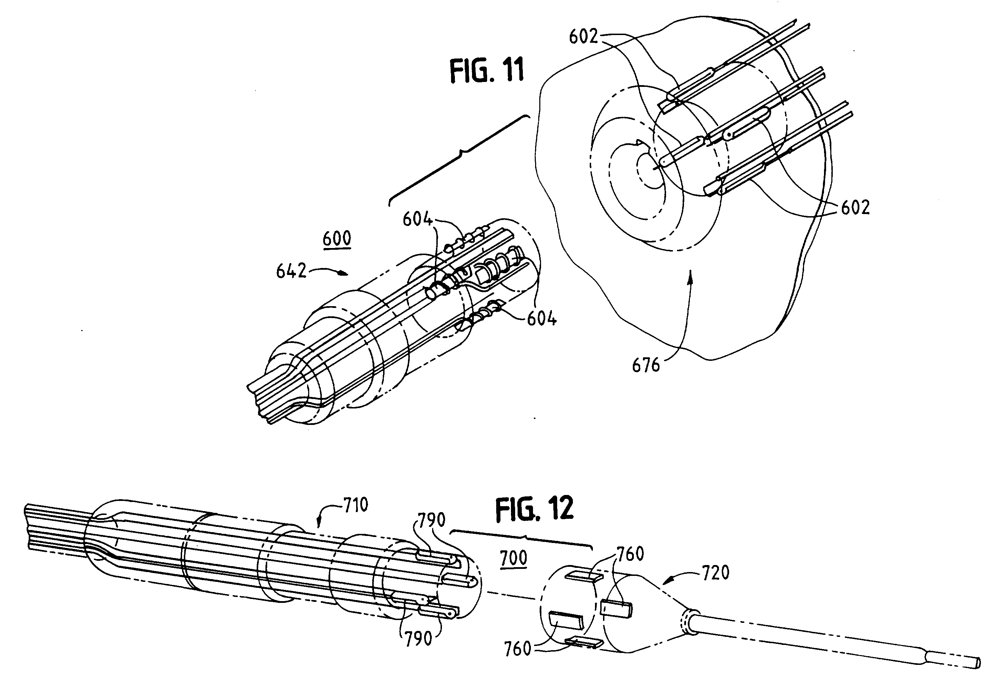

Referring now to FIG. 11, another embodiment of detection or identification apparatus of a surgical system 600 is illustrated. The surgical system 600 in many respects corresponds in construction and function to the surgical system 100 of FIG. 1 except that one or more reed switches 602 are arranged at predetermined positions on the receptacle assembly and electromagnets are arranged at predetermined positions on the plug assembly 642. Components of the present surgical system 600 which generally correspond to the components of the surgical system 100 of FIG. I are designated by like-numbers in the six-hundred series.

The detection apparatus of the surgical system 600 detects when the plug assembly 642 is attached to the generator unit 630 to detect or recognize the type or model of handpiece assembly 610. Energizing the electro-magnets 604, by power supplied from the generator unit 630, creates a localized magnet field which can change the condition of the reed switches 602 when the plug assembly 642 is inserted into the receptacle assembly 676. The change in condition of the switches 602 is detected by the control system of the generator unit 630 to provide appropriate power to the handpiece assembly 610 or to lock-out the handpiece assembly 610. Thus, if the magnets are provided in the intended configuration in the plug assembly, the generator unit will supply power to the transducer in the handpiece.

Referring to FIG. 12, therein is illustrated a further embodiment of the present surgical system, designated 700. The surgical system 700 is configured to identify or sense the type of surgical instrument 720 (which may comprise an ultrasonic blade and adaptor assembly) which is connected to an associated handpiece 710. To this end, at least one actuating device 760 is provided on the surgical instrument 720 for cooperation with at least one switch 790 provided on the handpiece assembly 710. In the illustrated embodiment, four actuating devices 760, preferably each comprising a permanent magnet, are provided for respective cooperation with a like member of switches 790.

It is contemplated that the arrangement of actuating devices and switches illustrated in FIG. 12 can be configured for cooperation with an arrangement of actuating devices and switches, such as generally described above, for the connection between the cable of the surgical instrument and the associated generator unit. By way of example, the connection between the cable and the generator can be provided with both permanent magnets and electro-magnets (on the cable connector). The permanent magnets cooperate with suitable switches on the connector receptacle in the generator to identify the handpiece assembly of the system. The electromagnets are operatively coupled to the switches 790 at the distal end of the handpiece assembly 710. The permanent magnets 760 on the blade and adaptor assembly 720 function to cooperate with the switches 790 when the blade assembly is joined to the handpiece so that the electromagnets at the cable connector are energized. These electromagnets then close additional reed switches in the connector receptacle in the generator to identify the blade fitted to the handpiece assembly.

The present invention provides methods and apparatus to detect when a connector of a surgical instrument is connected or attached to a connector of a generator or power supply unit and/or to identify or recognize the type of model of surgical instrument connected to the generator unit. After the surgical instrument is connected to the generator unit, the surgical instrument can be energized or can be prevent from being used with the generator unit.

Although the present invention has been described in detail by way of illustration and example, it should be understood that a wide range of changes and modifications can be made to the preferred embodiments described above without departing in any way from the scope and spirit of the invention. Thus, the described embodiments are to be considered in all aspects only as illustrative and not restrictive, and the scope of the invention is, therefore, indicated by the appended claims rather than the foregoing description. All changes that come within the meaning and range of equivalency of the claims are to be embraced within their scope.

## Claims

1. An ultrasonic surgical system comprising:
a handpiece assembly;
a transducer disposed in the handpiece assembly;
a cable assembly having a first end and a second end, the second end coupled to the handpiece assembly;
a first connector coupled to the first end of the cable assembly;
at least one actuating device coupled to the first connector;
a generator to provide energy to energize the transducer of the handpiece assembly, the generator having a second connector to receive the first connector;
at least one switching device coupled to the second connector; and
circuitry responsive to the switching device to detect when the first connector is coupled to the second connector.

2. The surgical system of claim 1 wherein the first connector comprises a male plug assembly, and the second connector comprises a female receptacle assembly.

3. The surgical system of claim 1 wherein the at least one switching device comprises one of a hall effect switch, a light emitting diode, a photo-detector, a micro-switch, a reed switch, a membrane switch, and a sensing device.

4. The surgical system of claim 1 wherein the at least one the actuating device comprises one of a permanent magnet and an electro-magnet.

5. The surgical system of claim 1 wherein the at least one switch opens to form an opened circuit when the first connector is coupled to the second connector.

6. An apparatus to identify surgical apparatus comprising:
a cable having a first end and a second end, the second end coupled to the surgical apparatus;
a plug assembly coupled to the first end of the cable, the plug assembly having at least one actuating device;
a receptacle assembly coupled to the first end of the cable to mate with the plug assembly, the receptacle assembly having at least one sensing device to change states between a first state and a second state when the receptacle assembly mates with the plug assembly; and
circuitry to detect the state of the at least one sensing device.

7. The apparatus of claim 6 wherein the at least one sensor device comprises a switch.

8. The apparatus of claim 6 wherein the circuitry includes a microprocessor.

9. The apparatus of claim 6 wherein the first connector comprises a male plug assembly, and the second connect comprises a female receptacle assembly.

10. The apparatus of claim 6 wherein the at least one switching device comprises one of a hall effect switch, a light emitting diode, a photo-detector, a micro-switch, a reed switch, a membrane switch, and a sensing device.
